# FASCICULE DE BREVET EUROPEEN

(11) **EP 0 840 626 B1**
(45) Date de publication et mention de la délivrance du brevet: **15.03.2000**
(21) Numéro de dépôt: 96926444.9
(22) Date de dépôt: 23.07.1996
(51) Int. Cl.: A61M 1/00, A61B 17/32

(54) **INSTRUMENT CHIRURGICAL DE DISSECTION A JET DE LIQUIDE SOUS HAUTE PRESSION**
CHIRURGISCHES DISSEKTIONSINSTRUMENT MIT HOCHDRUCKFLÜSSIGKEITSSTRAHL
SURGICAL DISSECTION INSTRUMENT USING A HIGH-PRESSURE LIQUID JET

(30) Priorité: 24.07.1995 FR 9509127
(43) Date de publication de la demande: 13.05.1998
(73) Titulaire: SAPHIR MEDICAL S.A., 69007 Lyon (FR)
(72) Inventeur: GONON, Bertrand, F-69002 Lyon (FR)
(74) Mandataire: Nithardt, Roland
(86) Numéro de dépôt international: FR9601154
(87) Numéro de publication internationale: WO9703713

(56) Documents cités:
- EP-A- 0 411 170
- WO-A-93/03777
- US-A- 3 713 443
- US-A- 4 519 385
- US-A- 5 147 292
- US-A- 5 191 881
- US-A- 5 254 117

## Description

La présente invention concerne un instrument chirurgical de dissection à jet de liquide sous haute pression, appelé aussi bistouri, comportant une pièce à main pourvue de moyens pour générer et contrôler un jet fin dudit liquide, constitué notamment de sérum physiologique, et un dispositif pour alimenter ladite pièce à main avec ce liquide sous haute pression.

On connaît de nombreux instruments de ce type appelés bistouris à jet de liquide. Un de ces appareils est par exemple décrit dans EP-A-0 411 170. Le préambule de la revendication 1 est basé sur ce document. L'instrument à main décrit dans EP-A-0 303 553 est équipé d'une vanne de commande manuelle qui permet d'arrêter le flux de liquide sous pression au cours d'une intervention chirurgicale. Habituellement, ces vannes sont de construction complexe du fait qu'elles comportent au moins une pièce mobile et un ressort de rappel agencé pour ramener la pièce mobile dans sa position initiale. En raison de cette relative complexité, la pièce à main devient une pièce coûteuse qui ne se prête pas à un usage unique. Or, les contraintes de stérilisation, qui sont imposées à ce type d'instruments, et les problèmes posés par la réutilisation, notamment en ce qui concerne les risques de contagion, imposent pratiquement le principe de l'usage unique.

Il existe déjà des pièces à main à usage unique utilisées dans des appareils d'irrigation ou de lavage des plaies et alimentées par un liquide sous faible pression, voire sous pression atmosphérique. De telles pièces à main sont décrites par exemple dans les publications américaines US-A-4 519 385 et US-A-5 147 292. Leur conception est totalement incompatible avec les contraintes techniques imposées dans un appareil destiné à disséquer des tissus et dans lequel la pression du liquide peut atteindre 70 bars.

De plus, la pièce à main doit avoir une forme ergonomique, tenir dans une seule main et comporter toutes les commandes de fonctions manoeuvrables aisément à l'aide des doigts de cette main. Cette commande doit être possible avec une main portant un gant de chirurgien, sans que le gant soit soumis à des contraintes risquant de le blesser. D'autre part, les doigts doivent pouvoir commander l'ouverture partielle ou totale et la fermeture du jet de liquide sous pression instantanément, sans effort important à fournir. La manoeuvre de commande doit être très souple et rapide compte tenu des contraintes de précision liées à l'utilisation d'un bistouri.

La fonction principale de la pièce à main est la commande de l'alimentation et de l'arrêt de l'alimentation en liquide sous haute pression.

Une fonction secondaire avantageuse est le verrouillage de la commande de l'alimentation en position d'arrêt.

Enfin, une troisième fonction est l'aspiration.

L'instrument chirurgical de dissection à jet de liquide sous haute pression, selon l'invention, telle que décrite dans la rev. 1 pallie les inconvénients de l'art antérieur et permet de remplir efficacement et à moindre coût la fonction principale, consistant à assurer l'ouverture et l'arrêt de l'alimentation en liquide. L'instrument comporte, entre autres, les caractéristiques suivantes:
- la pièce à main comporte un corps creux de forme générale allongée, ce corps s'étendant d'un embout proximal à un embout distal, les deux embouts étant situés sensiblement sur le même axe,
- ledit dispositif pour alimenter la pièce à main comporte un conduit souple logé dans ledit corps et s'étendant de l'embout distal jusqu'à l'embout proximal de façon sensiblement rectiligne, ledit conduit étant agencé pour résister à une haute pression au moins égale à 40 à 70 bars,
- l'embout proximal comporte une buse agencée pour générer ledit jet de liquide,
- lesdits moyens pour générer et contrôler ledit jet de liquide sous haute pression comportent :
   -- des moyens de pincement agencés pour écraser localement ledit conduit de manière à arrêter ledit jet de liquide sous haute pression et comportant, dans une zone proche de l'embout proximal, un plot de serrage fixe solidaire dudit corps, supportant ledit conduit et disposé perpendiculairement à ce dernier, et un plot de serrage mobile disposé en regard dudit plot fixe de l'autre côté dudit conduit perpendiculairement à ce dernier, les deux plots définissant entre eux un espace pour le passage dudit conduit, le plot mobile étant agencé pour se déplacer par rapport au plot fixe de manière à réduire ledit espace jusqu'à l'annuler assurant ainsi l'écrasement total dudit conduit dans une direction sensiblement perpendiculaire à ce dernier,
   -- une manette de commande desdits moyens de pincement agencée pour être manoeuvrée à la main par un utilisateur, montée dans un logement correspondant ménagé à la périphérie dudit corps et articulée sur un axe de pivotement dans ledit corps, cet axe de pivotement étant disposé entre l'embout proximal et le plot de serrage fixe, la manette de commande étant pourvue dudit plot de serrage mobile,
   -- des moyens de verrouillage associés à ladite manette de commande et agencés, en position verrouillée, pour bloquer ladite manette de commande dans une position basse dans laquelle le conduit est écrasé entre les deux plots de serrage et le jet de liquide sous haute pression est interrompu, et, en position déverrouillée, pour débloquer cette manette qui se soulève dans une position haute sous l'effet du jet de liquide sous haute pression qui ouvre le conduit.

Selon un mode de réalisation préféré, la pièce à main est réalisée dans une matière synthétique thermoplastique moulée par injection et le corps est constitué de deux demi-coques assemblées par emboîtement. Cette construction a l'avantage d'être très économique et permet de réaliser des pièces à usage unique, donc jetables.

De manière avantageuse, ladite manette de commande présente une forme générale rectangulaire et comporte une zone d'appui sensiblement plane ou incurvée agencée pour recevoir les doigts ou la paume de l'utilisateur. Elle est, plus particulièrement, articulée sur un pivot engagé dans une ouverture desdites demi-coques du corps de la pièce à main. Selon sa position, l'espace défini entre les deux plots de serrage est plus ou moins resserré.

Dans le mode de réalisation préféré, l'extrémité libre de ladite manette de commande définit avec le pivot un bras de levier D1 et le plot de serrage mobile définit avec ledit pivot un bras de levier D2 inférieur à D1, le rapport des bras de levier étant au moins compris entre 4 et 6.

Les moyens de verrouillage comportent, avantageusement, une languette souple solidaire du corps et portant un ergot d'accrochage agencé pour coopérer avec une griffe solidaire de la manette de commande. De préférence, cette languette souple comporte un poussoir ménagé à son extrémité supérieure et agencé pour libérer ladite manette de commande.

Selon le mode de réalisation préféré, l'instrument comporte un dispositif d'aspiration, un conduit souple traversant ladite pièce à main et un tube d'aspiration monté dans l'embout proximal.

La pièce à main comporte avantageusement une chambre disposée entre l'extrémité du conduit et le tube d'aspiration et au moins une ouverture latérale ménagée dans la paroi de la pièce à main et qui débouche dans ladite chambre.

Selon le mode de réalisation préféré, la pièce à main comporte deux ouvertures latérales correspondant à un canal central traversant partiellement ladite chambre, et un obturateur agencé pour obturer soit l'une soit l'autre desdites ouvertures. Cet obturateur peut comporter une partie centrale cylindrique et deux ailes latérales cannelées, sa longueur étant supérieure à la longueur de ladite pièce à main.

De préférence, la buse et le tube d'aspiration sont entourés d'une canule de protection montée sur l'embout proximal.

La présente invention sera mieux comprise en référence à la description d'une forme de réalisation préférée donnée à titre d'exemple non limitatif et représentée par les dessins annexés, dans lesquels :
- la figure 1 représente une vue en perspective de l'instrument à main, couplé à ses équipements périphériques représentés schématiquement,
- la figure 2 représente une vue en coupe axiale de l'instrument à main selon l'invention,
- les figures 3A et 3B représentent respectivement des vues intérieure et extérieure d'une demi-coque constitutive de la pièce à main,
- la figure 4 représente une vue en perspective d'une manette de commande de la pièce à main,
- les figures 5A et 5B sont des vues en coupe axiale illustrant respectivement la pièce à main en position de fermeture et en position d'ouverture, et
- la figure 6 représente un organe obturateur de la canule d'aspiration.

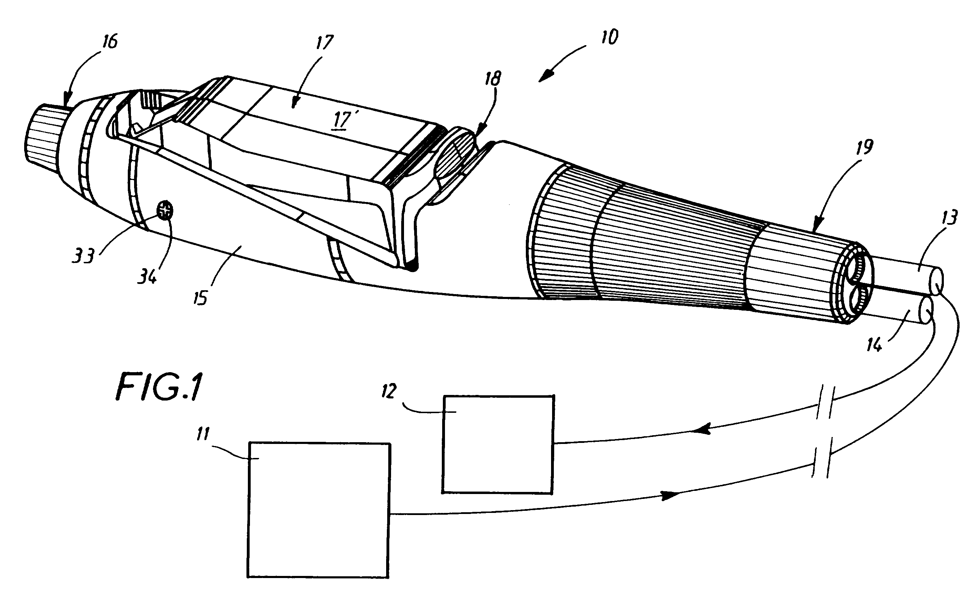

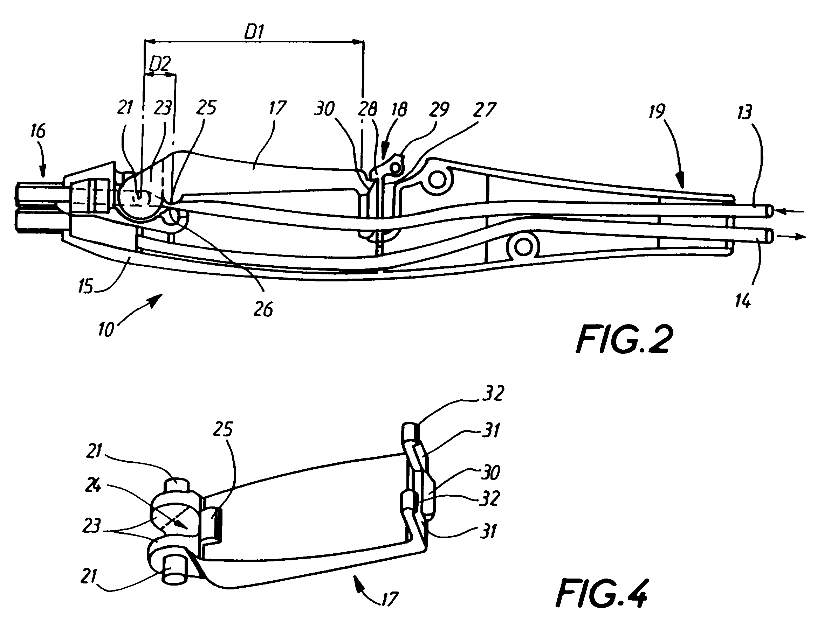

En référence aux figures, l'instrument chirurgical de dissection selon l'invention comporte une pièce à main 10, une source d'alimentation 11 en liquide physiologique sous haute pression et un dispositif d'aspiration 12. Ces équipements périphériques sont raccordés à la pièce à main par deux conduits souples respectivement 13 et 14 partiellement représentés sur les figures 1, 2, 5A et 5B. Ces conduits sont avantageusement renforcés par un blindage réalisé au moyen de fils synthétiques tressés, de manière à pouvoir résister à la pression du liquide qui peut atteindre 70 bars.

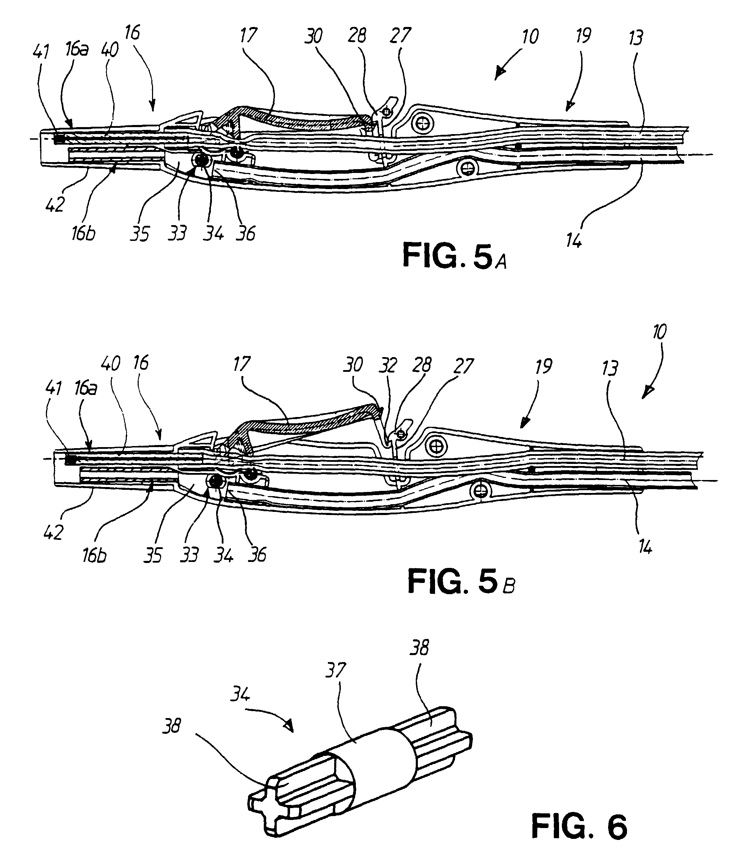

La pièce à main 10 est du type jetable, entièrement réalisée en matière synthétique thermoplastique moulée par injection. Elle comporte principalement un corps 15 ergonomique, de forme générale allongée, de section sensiblement circulaire, ce corps s'étendant d'un embout proximal 16 à un embout distal 19, les deux embouts étant situés sensiblement sur le même axe. En référence plus particulièrement aux figures 5A et 5B, l'embout proximal 16 est agencé pour recevoir une buse 16a générant le jet de liquide sous haute pression et disposée en prolongement du conduit 13 et un tube d'aspiration 16b canalisant l'aspiration et disposée en prolongement du conduit 14. La buse 16a est constituée d'une tige rigide 40 introduite à une de ses extrémités dans le conduit 13, traversée par un canal relativement étroit et pourvue à son autre extrémité d'un saphir 41 muni d'un orifice d'environ 0.1 mm apte à générer un jet de liquide très fin. La buse 16a et le tube d'aspiration 16b sont entourés d'une canule de protection 42 montée sur l'embout proximal 16. La buse 16a est en retrait par rapport à l'extrémité libre de la canule 42 et le tube d'aspiration 16b est en retrait par rapport à l'extrémité de la buse 16a. La canule 42 assure ainsi la protection des éléments qu'elle renferme mais aussi la protection de l'environnement extérieur proche contre les éventuelles projections de liquide. Elle est réalisée de préférence en matière synthétique très souple pour éviter de blesser les tissus. Cette canule 42 est sensiblement rectiligne comme dans les dessins ou de préférence coudée pour offrir une meilleure ergonomie. Dans ce cas, la tige 40 de la buse 16a est également coudée pour épouser la forme de la canule. L'embout distal 19 constitue une embouchure par où pénètrent les conduits 13 et 14 dans ledit corps. La pièce à main 10 comporte en plus une manette de commande 17, des moyens de pincement du conduit souple 13 et un organe de verrouillage 18 de la manette de commande en position fermée dans laquelle le conduit 13 est obturé et le jet de liquide sous haute pression arrêté.

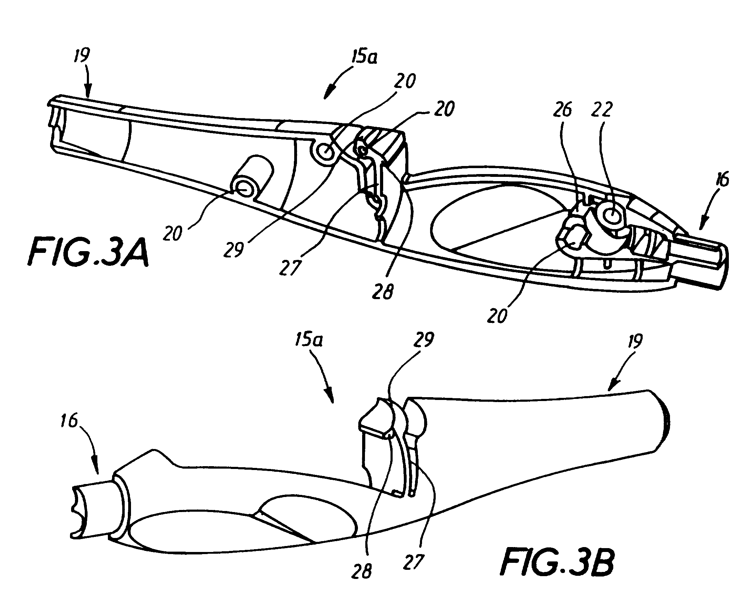

Le corps 15 est constitué de deux demi-coques moulées 15a, sensiblement identiques, dont l'une, la demi-coque femelle est représentée par les figures 3A et 3B. La demi-coque femelle diffère de la demi-coque mâle par l'absence d'ergots d'assemblage par clipsage et par la présence de logements 20 agencés pour recevoir ces ergots. Les deux demi-coques sont intérieurement creuses pour permettre le passage des conduits 13 et 14 à l'intérieur du corps de la pièce à main depuis l'embout distal 19 jusqu'à l'embout proximal 16 selon un trajet sensiblement rectiligne.

La manette de commande 17 présente une forme générale rectangulaire et comporte une zone d'appui 17' sensiblement plane ou incurvée agencée pour recevoir les doigts ou la paume de l'utilisateur. Elle est située dans le prolongement dudit corps 15 et articulée sur un pivot 21 engagé dans une ouverture 22 des demi-coques du corps de la pièce à main dans une zone proche de l'embout proximal. Ce pivot se compose en fait de deux éléments symétriques dont chacun est solidaire d'une oreille 23, les deux oreilles étant disposées parallèlement entre elles. Ces oreilles 23 ménagent un passage 24 pour le conduit d'alimentation 13 en liquide physiologique sous haute pression. Dans le prolongement de ce passage, est disposé un plot de serrage mobile 25 solidaire de la manette de commande 17, ce plot de serrage mobile 25 étant positionné en regard d'un plot de serrage fixe 26 solidaire du corps de la pièce à main et agencé pour coopérer avec ce dernier pour écraser le conduit 13, lorsque l'utilisateur appuie sur la manette de commande 17 et arrêter instantanément le flux de liquide sous haute pression. Plus précisément, les plots de serrage 25, 26 définissent entre eux un espace pour le passage du conduit 13 et sont disposés perpendiculairement à ce dernier. Le plot de serrage mobile 25 est agencé pour se déplacer par rapport au plot de serrage fixe 26 de manière à réduire ledit espace jusqu'à l'annuler assurant ainsi l'écrasement total dudit conduit 13 dans une direction sensiblement perpendiculaire à ce dernier. L'extrémité libre de la manette de commande 17 définit avec le pivot 21 un bras de levier D1 et le plot de serrage mobile 25 définit avec le pivot 21 un bras de levier D2 très inférieure à D1. Dans l'exemple illustré, le rapport des bras de levier D1, D2 est proche de 5. En conséquence, l'effort de manoeuvre à appliquer sur la manette de commande 17 est divisé par cinq. Cette construction originale permet de comprimer le conduit 13 plus ou moins, même sous très forte pression, en manoeuvrant la manette avec les doigts d'une main aisément, de manière souple et sans fournir un effort important.

L'organe de verrouillage 18 se compose principalement d'une languette souple 27 solidaire du corps 15 de la pièce à main et disposée dans un plan sensiblement perpendiculaire à l'extrémité libre de la manette de commande 17. Cette languette porte un ergot d'accrochage 28 proche de son extrémité supérieure qui est équipée d'un poussoir 29. L'ergot d'accrochage 28 est agencé pour coopérer avec une griffe 30 solidaire de la manette de commande 17, lorsque l'utilisateur enfonce suffisamment ladite manette en vue de provoquer l'arrêt du flux de liquide physiologique sous haute pression par pincement du conduit souple 13. Pour décrocher la manette 17, il agit sur le poussoir 29, à l'aide de son pouce, ce qui a pour effet de dégager la griffe 30 de l'ergot 28. La flexibilité du conduit souple 13 et la pression du liquide physiologique véhiculé par ce conduit ramènent la manette de commande 17 dans sa position initiale dans laquelle le conduit 13 est totalement ouvert.

La griffe 30 occupe une position centrale entre deux pattes 31 munies chacune d'un bec 32. Les becs 32 sont agencés pour coopérer avec l'ergot d'accrochage 28 de la languette souple 27 pour définir une position de blocage de la manette de commande 17 ouverte, telle qu'illustrée par la figure 5B et dans laquelle le conduit 13 est ouvert complètement.

Comme le montrent plus spécifiquement les figures 5A et 5B, la pièce à main est à deux positions, une position de fermeture (fig. 5A) et une position d'ouverture (fig. 5B). Dans la position de fermeture, la griffe 30 solidaire de la manette de commande 17 est retenue par l'ergot d'accrochage 28. La manette est enfoncée et le conduit 13 est obturé par pincement entre le plot de serrage 25 et l'arrêt fixe 26. Dans la position d'ouverture (fig. 5B), les becs 32 solidaires des pattes 31 sont retenus par l'ergot d'accrochage 28. La manette 17 est relevée et le conduit 13 est desserré, pour permettre le passage du liquide sous haute pression. Entre ces deux positions, la manette 17 peut être placée dans une position intermédiaire à partir de laquelle le conduit 13 est obturé alors que la manette 17 n'est pas verrouillée par la languette 27. Cette position intermédiaire permet d'interrompre momentanément le flux du liquide sous pression tout en maintenant l'aspiration.

Sur le flanc de la pièce à main 10 apparaît une ouverture latérale 33 qui correspond à un canal traversant dans lequel est logé un obturateur 34 représenté par la figure 6. Le canal débouche dans une chambre 35 raccordée à l'extrémité 36 du conduit d'aspiration 14 connecté à une pompe à vide. L'obturateur 34 est agencé pour coulisser à l'intérieur du canal traversant et comporte de part et d'autre d'une partie centrale cylindrique 37, deux ailes latérales 38 cannelées formant avec l'une ou l'autre des ouvertures latérales 33 des orifices de communication entre ladite chambre d'aspiration 35 et l'environnement extérieur de la pièce à main. La longueur totale de l'obturateur 34 est supérieure à la largeur de la pièce à main de sorte que le chirurgien peut choisir d'obturer l'une ou l'autre ouverture latérale 33 en faisant coulisser l'obturateur 34 à l'intérieur dudit canal traversant le corps de la pièce à main. Lorsque le chirurgien obture, au moyen de son index gauche, s'il est gaucher, ou droit, s'il est droitier, une des ouvertures 33, le conduit 14 est raccordé à la canule 16b d'aspiration à travers la chambre 35. Si le chirurgien enlève son index de l'ouverture 33, l'effet de succion au niveau de la canule d'aspiration s'arrête, l'aspiration se faisant à travers l'ouverture 33. Grâce à cet obturateur, la pièce à main est rendue universelle, adaptée à la fois aux droitiers et aux gauchers.

A l'utilisation, la pièce à main 10 se tient et se manipule aisément d'une seule main. Le corps 15 de cette pièce présente deux flancs concaves qui lui donnent un profil ergonomique adapté aux doigts du chirurgien. La manette de commande 17 se manipule de manière souple à l'aide des doigts de la main. Il en est de même de l'organe de verrouillage 18. Sa conception est simple et sa construction en fait un composant peu coûteux à la fabrication, de sorte qu'il peut être considéré et utilisé comme pièce à usage unique.

Dans la pratique, la pièce à main 10 et les conduits 13 et 14 sont conditionnés dans un sachet stérile. Les autres pièces stériles et jetables nécessaires pour mettre en oeuvre l'instrument chirurgical de dissection décrit, telles que la poche de sérum physiologique et l'ensemble de perfusion reliant ladite poche au conduit 13 de la pièce à main, sont également conditionnées dans des sachets individuels stériles.

Il va de soi que ce principe d'aspiration et de commande de l'arrêt de la succion pourrait être également mis en oeuvre avec une seule ouverture latérale 33 ménagée dans le corps de la pièce à main. Dans ce cas, l'obturateur 34 n'a plus de raison d'exister.

La présente invention n'est pas limitée aux formes de réalisation décrites, mais peut subir différentes variantes et se présenter sous divers aspects dérivant des formes décrites d'une manière évidente pour un homme du métier.

## Revendications

1. Instrument chirurgical de dissection à jet de liquide sous haute pression, appelé bistouri, comportant une pièce à main pourvue de moyens pour générer et contrôler un jet dudit liquide sous haute pression, constitué notamment de sérum physiologique, et un dispositif pour alimenter ladite pièce à main avec ce liquide sous haute pression, dans lequel :
- la pièce à main (10) comporte un corps creux (15) de forme générale allongée, ce corps s'étendant d'un embout proximal (16) à un embout distal (19), les deux embouts étant situés sensiblement sur le même axe,
- ledit dispositif pour alimenter la pièce à main (10) comporte un conduit souple (13) logé dans ledit corps (15),
- l'instrument comporte une buse (16a) agencée pour générer ledit jet de liquide,
- lesdits moyens pour générer et contrôler ledit jet de liquide sous haute pression comportent :
-- des moyens de pincement agencés pour écraser localement ledit conduit (13) de manière à arrêter ledit jet de liquide sous haute pression et comportant un plot de serrage fixe (26) solidaire dudit corps (15), supportant ledit conduit (13) et disposé perpendiculairement à ce dernier, et un plot de serrage mobile (25) disposé en regard dudit plot fixe (26) de l'autre côté dudit conduit (13) perpendiculairement à ce dernier, les deux plots (25, 26) définissant entre eux un espace pour le passage dudit conduit (13), le plot mobile (25) étant agencé pour se déplacer par rapport au plot fixe (26) de manière à réduire ledit espace jusqu'à l'annuler assurant ainsi l'écrasement total dudit conduit (13) dans une direction sensiblement perpendiculaire à ce dernier,
-- une manette de commande (17) desdits moyens de pincement agencée pour être manoeuvrée à la main par un utilisateur articulée sur un axe de pivotement disposé entre l'embout proximal (16) et le plot de serrage fixe (26), la manette de commande (17) étant pourvue dudit plot de serrage mobile (25),
caractérisé en ce que: le conduit souple (13) s'étend de l'embout distal (19) jusqu'à l'embout proximal (16) de façon sensiblement rectiligne, ledit conduit (13) étant agencé pour résister à une haute pression au moins égale à 40 bars, la buse (16a) est agencée à l'embout proximal (16), les plots de serrage (26,25) sont disposés dans une zone proche de l'embout proximal (16), la manette de commande (17) est montée dans un logement correspondant ménagé à la péripherie dudit corps (15) et l'axe de pivotement est logé dans ledit corps, ledits moyens pour générer et contrôler ledit jet de liquide sous pression comportant en outre:
-- des moyens de verrouillage (18) associés à ladite manette de commande (17) et agencés, en position verrouillée, pour bloquer ladite manette de commande (17) dans une position basse dans laquelle le conduit (13) est écrasé entre les deux plots de serrage (25, 26) et le jet de liquide sous haute pression est interrompu, et, en position déverrouillée, pour débloquer cette manette qui se soulève dans une position haute sous l'effet du jet de liquide sous haute pression qui ouvre le conduit (13).

2. Instrument selon la revendication 1, caractérisé en ce que la pièce à main (10) est réalisée dans une matière synthétique thermoplastique moulée par injection et en ce que ledit corps (15) est constitué de deux demi-coques (15a) assemblées par emboîtement.

3. Instrument selon la revendication 1, caractérisé en ce que la manette de commande (17) présente une forme générale rectangulaire et comporte une zone d'appui (17') sensiblement plane ou incurvée agencée pour recevoir les doigts ou la paume de l'utilisateur.

4. Instrument selon la revendication 3, caractérisé en ce que ladite manette de commande (17) est articulée sur un pivot (21) engagé dans une ouverture (22) desdites demi-coques (15a) du corps de la pièce à main (10) et ce que, selon la position de ladite manette de commande (17), l'espace défini entre les deux plots de serrage (25, 26) est plus ou moins resserré.

5. Instrument selon la revendication 4, caractérisé en l'extrémité libre de la manette de commande (17) définit avec le pivot (21) un bras de levier D1, en ce que le plot de serrage (25) définit avec le pivot (21) un bras de levier D2 inférieur à D1 et en ce que le rapport des bras de levier D1/D2 est au moins compris entre 4 et 6.

6. Instrument selon la revendication 1, caractérisé en ce que les moyens de verrouillage (18) comportent une languette souple (27) solidaire du corps (15) et portant un ergot d'accrochage (28) agencé pour coopérer avec une griffe (30) solidaire de la manette de commande (17).

7. Instrument selon la revendication 6, caractérisé en ce que ladite languette souple (27) comporte un poussoir (29) ménagé à son extrémité supérieure et agencé pour libérer ladite manette de commande (17).

8. Instrument selon la revendication 1, caractérisé en ce qu'il comporte un dispositif d'aspiration (12), un conduit souple (14) traversant ladite pièce à main (10) et un tube d'aspiration (16b) monté dans l'embout proximal (16).

9. Instrument selon la revendication 8, caractérisé en ce que la pièce à main (10) comporte une chambre (35) disposée entre l'extrémité (36) du conduit (14) et le tube d'aspiration (16b) et au moins une ouverture latérale (33) ménagée dans la paroi de la pièce à main et débouchant dans ladite chambre (35).

10. Instrument selon la revendication 9, caractérisé en ce que la pièce à main (10) comporte deux ouvertures latérales (33) correspondant à un canal central traversant partiellement ladite chambre (35), et un obturateur (34) agencé pour obturer soit l'une soit l'autre desdites ouvertures (33).

11. Instrument selon la revendication 10, caractérisé en ce que l'obturateur (34) comporte une partie centrale cylindrique (37) et deux ailes latérales (38) cannelées et en ce que sa longueur est supérieure à la largeur de ladite pièce à main.

12. Instrument selon la revendication 8, caractérisé en ce que la buse (16a) et le tube d'aspiration (16b) sont entourés d'une canule de protection (42) montée sur l'embout proximal (16).

## Patentansprüche

1. Skalpell benanntes chirurgisches Dissektionsinstrument mit Hochdruckflüssigkeitsstrahl, bestehend aus einem mit Mitteln zur Erzeugung und Verstellung eines Hochdruckstrahls der besagten Flüssigkeit, insbesondere physiologische Kochsalzlösung, ausgestatteten Handstück, sowie aus einer Vorrichtung zur Versorgung des besagten Handstücks mit dieser Hochdruckflüssigkeit, in welchem :
- das Handstück (10) einen hohlen Körper (15) mit einer generellen länglichen Form beträgt, wobei dieser Körper sich von einem nahen Endstück (16) bis zu einem fernen Endstück (19) erstreckt, wobei sich beide Endstücke merklich auf der selben Achse befinden, die besagte Vorrichtung zur Versorgung des Handstücks (10) eine in dem besagten Körper (15) untergebrachte flexible Leitung (13) aufweist, und das Instrument eine zur Erzeugung des besagten Flüssigkeitsstrahls angeordnete Düse (16a) aufweist die besagten Mittel zur Erzeugung und Verstellung des besagten Hochdruckstrahls aus folgenden Elementen bestehen- Quetschmittel, die angeordnet sind, um die besagte Leitung (13) lokal zu quetschen um den besagten Hochdruckflüssigkeitsstrahl zu unterbrechen, und die ein fester, aus einem Stück mit dem besagten Körper (15) hergestellter Quetschnocken (26) aufweisen, welcher die besagte Leitung (13) trägt und quer zu dieser angeordnet ist, sowie ein gegenüber des besagten festen Quetschnockens (26), auf der anderen Seite der besagten Leitung quer zu dieser angeordneter beweglicher Quetschnocken (25), wobei beide Quetschnocken (25, 26) zwischen sich einen Freiraum für den Durchgang der besagten Leitung (13) bilden, wobei der bewegliche Quetschnocken (25) angeordnet ist um sich in Bezug auf den festen Quetschnocken (26) zu bewegen und so den besagten Freiraum bis zu dessen Verschwinden zu verringern, wodurch das vollständige Quetschen der besagten Leitung (13) in eine zu dieser merklich rechtwinkligen Richtung erfolgt,
- ein Steuerhebel (17) der besagten Quetschnocken, welcher angeordnet ist um von Hand durch den Benutzer betätigt zu werden, auf einer Drehachse drehend und zwischen dem nahen Endstück (16) und dem festen Quetschnocken (26) angeordnet, wobei der besagte Steuerhebel (17) mit dem besagten beweglichen Quetschnocken (25) versehen ist, gekennzeichnet dadurch, daß die flexible Leitung (13) sich merklich geradlinig von dem fernen Endstück (19) bis zum nahen Endstück (16) erstreckt, wobei die besagte flexible Leitung angeordnet ist, um einen Hochdruck von mindestens 40 Bar standzuhalten, dadurch, daß die Düse (16a) am Ende des nahen Endstücks (16) angeordnet ist, dadurch, daß sich die Quetschnocken (26, 25) in einem Bereich in der Nähe des nahen Endstücks (16) befinden, dadurch, daß der Steuerhebel (17) in einer entsprechenden, auf der Peripherie des besagten Körpers (15) vorgesehenen Aufnahme montiert ist und daß die Drehachse in dem besagten Körper untergebracht ist, wobei die Mittel zur Erzeugung und zur Verstellung des besagten Hochdruckflüssigkeitsstrahls außerdem folgende Elemente betragen:
- mit dem besagten Steuerhebel (17) zusammenwirkende Verriegelungsmittel (18) die, im verriegelten Zustand, angeordnet sind um den besagten Steuerhebel (17) in einer unteren Position festzuhalten, in welcher die Leitung (13) zwischen den beiden Quetschnocken (25, 26) zusammengequetscht ist und der Hochdruckflüssigkeitsstrahl unterbrochen ist, und die, im entriegelten Zustand, angeordnet sind um diesen Steuerhebel zu befreien, wodurch der Druck des Flüssigkeitsstrahls die Leitung (13) öffnet und den Steuerhebel in die obere Position drückt.

2. Instrument gemäß Anforderung 1, gekennzeichnet dadurch, daß das Handstück (10) aus einem thermoplastischen Kunststoff durch ein Spritzverfahren hergestellt wird, und daß der besagte Körper (15) aus zwei durch Einrasten zusammengebaute Halbschalen (15a) besteht.

3. Instrument gemäß Anforderung 1, gekennzeichnet dadurch, daß der Steuerhebel (17) eine generelle rechteckige Form aufweist und eine merklich flache oder abgerundete Auflagefläche (17') beträgt, die angeordnet ist um die Finger oder die Handfläche des Benutzers aufzunehmen.

4. Instrument gemäß Anforderung 3, gekennzeichnet dadurch, daß der besagte Steuerhebel (17) auf einer Drehachse (21) dreht, die in einer Öffnung (22) in beiden Halbschalen (15a) des Körpers des Handstücks (10) eingefügt ist, und dadurch, daß, je nach der Position des besagten Steuerhebels (17), der zwischen den beiden Quetschteilen (25, 26) entstehende Freiraum mehr oder weniger eng ist.

5. Instrument gemäß Anforderung 4, gekennzeichnet dadurch, daß das freie Ende des Steuerhebels (17) mit der Drehachse (21) einen Hebelarm D1 bildet, dadurch, daß der Quetschnocken (25) mit der Drehachse (21) einen Hebelarm D2 bildet, der kleiner als D1 ist, und dadurch, daß das Hebelarm-Verhältnis D1/D2 mindestens zwischen 4 und 6 liegt.

6. Instrument gemäß Anforderung 1, gekennzeichnet dadurch, daß die Verriegelungsmittel (18) einen aus einem Stück mit dem Körper (15) hergestellten elastischen Lappen (27) betragen, der einen Nocken (28) trägt, welcher angeordnet ist, um mit einer aus einem Stück mit dem Steuerhebel (17) hergestellte Klaue (30) zusammenzuarbeiten.

7. Instrument gemäß Anforderung 6, gekennzeichnet dadurch, daß der besagte elastische Lappen (27) eine an seinem oberen Ende befindlichen Druckfläche (29) beträgt, die angeordnet ist, um den besagten Steuerhebel (17) zu befreien.

8. Instrument gemäß Anforderung 1, gekennzeichnet dadurch, daß er eine Absaugvorrichtung (12), eine durch das besagte Handstück (10) gehende flexible Leitung (14) und ein im den nahen Endstück (16) montiertes Absaugrohr (16b) beträgt.

9. Instrument gemäß Anforderung 8, gekennzeichnet dadurch, daß das Handstück (10) eine zwischen dem Ende (36) der Leitung (14) und dem Ansaugrohr (16b) angeordnete Kammer (35), sowie mindestens eine in der Wand des Handstücks angeordnete und in der besagten Kammer (35) endende seitliche Öffnung (33) beträgt.

10. Instrument gemaß Anforderung 9, gekennzeichnet dadurch, daß das Handstück (10) zwei seitliche Öffnungen (33), die einem mittleren Kanal entsprechen, der zum Teil durch die besagte Kammer (35) geht, sowie einen Verschluß (34) beträgt, wobei letzterer angeordnet ist um entweder die eine oder die andere der besagten Öffnungen (33) zu verschließen.

11. Instrument gemäß Anforderung 10, gekennzeichnet dadurch, daß der Verschluß (34) ein zylindrisches Mittelteil (37) und zwei geriefte Seitenteile (38) aufweist, und dadurch, daß seine Länge größer ist, als die Breite des besagten Handstücks.

12. Instrument gemäß Anforderung 8, gekennzeichnet dadurch, daß die Düse (16a) und das Ansaugrohr (16b) von einer auf dem nahen Ende (16) angebrachten Schutzkanüle (42) umgeben sind.

## Claims

1. Surgical dissection instrument using a jet of high-pressure liquid, called a lancet, comprising a handpiece provided with means of generating and controlling a jet of said high-pressure liquid, notably made up of physiological salt solution, and a device for supplying said handpiece with this high-pressure liquid in wich :
- the handpiece (10) comprises a hollow body (15) of generally elongated shape, this body extending from a near end-cap (16) to a far end-cap (19), both ends being located significantly on the same axis,
- said device for supplying the handpiece (10) comprises a flexible tube (13) housed in said body (15),
- the instrument comprises a nozzle (16a) designed to generate said liquid jet,
- said means of generating and controlling said high-pressure liquid jet comprise :
- pinching means, designed to locally squash said tube (13) so as to stop said high-pressure liquid jet and comprising a fixed squeezing projection (26) secured to said body (15), supporting said tube (13) and arranged perpendicular to the latter, and a movable squeezing projection (25) arranged opposite said fixed projection (26) on the other side of said tube (13) perpendicular to the latter, the two projections (25, 26) defining between them a space for said tube (13) to pass, the movable projection (25) being designed to move in relation to the fixed projection (26) so as to reduce said space until it is cancelled, thus ensuring that said tube (13) is totally squashed in a significantly perpendicular direction to the latter,
- a lever (17) for controlling said pinching means designed to be hand-operated by a user, articulating on a swivel pin located between the near end-cap (16) and the fixed squeezing projection (26), the control lever (17) being provided with the said movable squeezing projection (25); characterized in that the flexible tube (13) extending from the far end-cap (19) to the near end-cap (16) in a significantly rectilinear manner, said tube (13) being designed to withstand a high pressure of at least 40 bars, the nozzle (16a) is disposed near the end-cap (16), the squeezing projections (25, 26) are set in an area close to the near end-cap (16), the lever (17) is mounted in a corresponding housing located in the periphery of said body (15) and the swivel pin is located in said body, said means of generating and controlling said high-pressure comprising also :
- locking means (18) combined with said control lever (17) and designed, in the locked position, to block said control lever (17) in a low position in which the tube (13) is squashed between the two squeezing projections (25, 26) and the high-pressure liquid jet is cut off, and, in the unlocked position, to release this lever which rises into a high position under the effect of the high-pressure liquid jet which opens the tube (13).

2. Instrument according to claim 1, characterized in that the handpiece (10) is made of an injection-moulded thermoplastic synthetic material and in that said body (15) is made up of two half shells (15a) assembled by slotting them together.

3. Instrument according to claim 1, characterized in that said control lever (17) has a general rectangular shape and comprises a significantly flat or inwardly curved supporting area (17') to receive the user's fingers or palm.

4. instrument according to claim 3, characterized in that said control lever (17) is articulated on a pivot (21) engaged in an opening (22) in said half shells (15a) of the handpiece's (10) body, and in that depending on the position of said control lever (17), the space defined between the two squeezing projections (25, 26) is more or less confined.

5. Instrument according to claim 4, characterized in that the free end of said control lever (17) defines with the pivot (21) a lever arm D1 and in that the squeezing projection (25) defines with the pivot (21) a lever arm D2 smaller than D1, and in that the ratio of the lever arms is at least between 4 and 6.

6. Instrument according to claim 1, characterized in that the locking means (18) comprise a flexible tongue (27) secured to the body (15) and bearing a catch pin (28) designed to cooperate with a grip (30) secured to the control lever (17).

7. Instrument according to claim 6, characterized in that said flexible tongue (27) comprises a pusher (29) located at its upper end and designed to release said control lever (17).

8. Instrument according to claim 1, characterized in that it comprises a suction device (12), a flexible tube (14) crossing said handpiece (10) and a suction tube (16b) mounted in the near end-cap (16).

9. Instrument according to claim 8, characterized in that the handpiece (10) comprises a chamber (35) located between the end (36) of the tube (14) and the suction tube (16b) and at least one lateral opening (33) provided in the wall of the handpiece which joins said chamber (35).

10. Instrument according to claim 9, characterized in that the handpiece (10) comprises two lateral openings (33) corresponding to a central channel partially crossing said chamber (35), and a seal (34) designed to seal either of said openings (33).

11. Instrument according to claim 10, characterized in that the seal (34) has a cylindrical central part (37) and two grooved lateral wings (38), and in that its length is greater than the length of said handpiece.

12. Instrument according to claim 8, characterized in that the nozzle (16a) and the suction tube (16b) are encompassed by a protective cannula (42) mounted on the near end-cap (16).
